# EUROPEAN PATENT APPLICATION

(11) **EP 3 862 418 A1**
(43) Date of publication of application: **11.08.2021**
(21) Application number: 20156005.9
(22) Date of filing: 07.02.2020
(51) Int. Cl.: C12M 1/42, C12N 13/00

(54) **DEVICE FOR TREATING CELLS**

(71) Applicant: Bühler AG, 9240 Uzwil (CH)
(72) Inventor: LARSEN, Sara, Newport, Essex, CB11 3RN (GB); O'REILLY, John, Co. Cork (IE); MATHYS, Alexander, 8044 Zürich (CH); BUCHMANN, Leandro, 8408 Winterthur (CH); GEORGET Erika, 8041 Zürich (CH); BUENDER, Jana Carmen, 6283 Baldegg (CH)
(74) Representative: Hepp Wenger Ryffel AG

(57) **Abstract**

The present invention relates to a device (1) for treating cells for stimulating cell growth, comprising a unit (2) for generating and emitting electric pulses and an electric field resulting therefrom, and a treatment space (3) that can be penetrated by the emitted electric pulses, wherein the device (1) further comprises a unit (4) which can be moved into and out of said treatment space (3) such that cell material provided within a compartment (4a) of said unit (4), or provided in a container (5) on said unit (4), can be moved into and out of said treatment space (3).

## Description

The present invention relates to a device for treating cells used in medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds.

It is known that prokaryotic and eukaryotic cells are influenced by the action of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265).

EP-2 308 969 B1 describes a PEF (pulsed electric field) method where a cell material suspended in an electrically conductive liquid, the cell material being positioned between two electrodes, by exposure of 1 to 100 electric pulses, such that a voltage increase takes place between the two electrodes of 10% to 90% of a target voltage of the electric pulses within a period of 0.1 to 100 ns, the electric pulses have a pulse duration of 5 ns to 5000 ns, and the electric pulses, upon reaching the target voltage, have an electric field strength of 0.5 kV/cm to 50 kV/cm, showed an accelerated cell proliferation and/or increased cell constituents.

In the device for treating cell material used in EP 2 308 969 B1, an electroporation cuvette having the suspension of cell material described above was continuously pumped through an arrangement of two electrodes and exposed to an electric field in an electrically conductive liquid (paragraph [0020] of EP-2 308 969 B1).

Under these conditions, a flow profile is formed between the wall and the center of the vessel, which results in deviations with respect to the process conditions over the vessel diameter. This is a problem since influencing the cell proliferation as described above depends on the accurate maintenance of the process conditions. With the device of EP-2 308 969 B1, it is not possible to obtain homogeneous process conditions in the entire reaction vessel and therefore also no homogeneously influenced cell material.

It was the object of the present invention to overcome the above problems of the prior art and to provide a device that overcomes difficulties involved with currently available PEF equipment in order to obtain homogenous and controllable flow and electric field profiles.

The above object is achieved by the present invention as defined in the claims.

In detail, the present invention relates to a device, comprising a unit for generating and emitting electric pulses, a treatment space capable of being penetrated by the emitted electric pulses and an electric field resulting therefrom, wherein the device further comprises a unit which can be moved into and out of said treatment space such that cell material provided within a compartment of said unit, or provided in a container on said unit, can be moved into and out of said treatment space.

According to the present invention, the device is suitable for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

According to the present invention, an electrical field is applied to a cell material located in a compartment of a unit or in a container on the unit. As a result, the cell material is subjected to the electric field from a uniaxial direction, and a homogeneous stimulation of cell growth is achieved.

The concept of the present invention is that by movement of a unit, a compartment in said unit or a container provided on said unit is moved into a certain region of the device, which is called treatment space. Once the respective compartment or container has been moved into the treatment space, an electric field acts on the cell material provided in said compartment or container. Preferably, said movement of the unit is a rotational movement.

According to the present invention, a rotational movement is understood to mean a partial or complete rotation about an imaginary axis. The partial rotation should be at least 180°, wherein a rotation in the range of 360° is particularly preferred.

According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells. Furthermore, multicellular tissues such as meristems in plants and epithelial or connective tissue in humans or animals can be treated.

The cell material is usually (but not necessarily) isolated, purified and/or sterilized in a known manner before being treated according to the present invention. Preferably, the cell material can already be propagated in a known manner in suitable and known culture media to a desired degree before the treatment according to the present invention.

The cell material is preferably suspended in an electrically conductive liquid prior to the treatment according to the present invention. Electrically conductive liquids are well known. According to the present invention, it is necessary to use electrically conductive liquids which have no adverse effects on cell viability, that is, in particular, are non-toxic. According to the present invention, water is preferably used as the electrically conductive liquid, wherein the water can be adjusted to a desired pH value by means of suitable and known additives. According to the present invention, a pH value in the range of 6.0 to 14.0, preferably 7 to 12 is preferred.

According to the present invention, the suspensions described above can be prepared in a conventional manner and stored until treatment. However, the suspensions can also be provided immediately before the treatment according to the present invention.

According to the present invention, conventional containers can be used for receiving the cell material. Examples would be bottles, flasks, test tubes, or cuvettes.

According to a preferred embodiment of the present invention, compartments provided in the unit are of a cylindrical shape. However, also other forms of compartments may be used.

Units for generating and emitting electric pulses are well known. According to the present invention, devices which can generate electrical impulses as described below are preferred. Such devices are known. By way of example, cable pulse generators, semiconductor-based pulse generators, or relaxation oscillators can be mentioned.

The generated electric pulses are emitted in a treatment space. This is preferably done by two or more electrodes or plates of a capacitor arranged parallel to each other, which are arranged opposite each another having a distance suitable for the generation of electric pulses. Such arrangements are well known and need not be explained in detail here.

According to a preferred embodiment of the present invention, two electrodes or plates of a capacitor are arranged perpendicularly to the direction of movement of the unit in or on which the compartment or container is provided. When the compartment or container enters the treatment space, it takes a position in the space between said electrodes or plates, so that one electrode/plate is provided over said compartment or container, and the other electrode or plate is provided below said compartment or container.

The electric field to be applied to the treatment space must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

The compartment or container containing the cell material to be treated is arranged, according to the present invention, in the treatment space provided between the two electrodes or plates of a capacitor parallel to each other. This arrangement is carried out by subjecting the unit in which the compartments are provided, or on which the containers are provided, to a, preferably rotational, movement. The manner of generating the, preferably rotational, movement and the applied speed of movement is not subject to any particular limitation.

According to preferred embodiments of the present invention, the preferably rotational, movement of the unit can be realized by arranging said unit on a motor for generating a rotational movement.

According to a first embodiment, the unit is a body, preferably a cylindrical body, which has at least one compartment for receiving the cell material and is arranged on a motor for generating a rotational movement.

Such units are known. The compartments are preferably blind holes having suitable dimensions.

It is preferred according to this embodiment that 1 to 20, preferably 2 to 15, more preferably 5 to 10 compartments can be arranged in the unit.

According to a preferred embodiment of the present invention, the at least one compartment is filled with cell material by means of a filling unit. Said filling unit comprises an outlet through which cell material can be inserted into the at least one compartment.

The outlet of the filling unit is preferably connected to a device in which the cell material or a suspension containing the cell material is located. For example, conventional pipelines can be provided for this connection. Preferably, a shut-off unit such as a valve or a lock is provided in or at the outlet or alternatively in the connection described above in order to be able to control the introduction of the cell material or the suspension containing the cell material into the compartment.

The outlet of the filling unit is preferably located in a region of the device remote from the treatment space, here designated as filling region. Preferably, the filling region is arranged such that the compartment is first moved into the filling region, where the at least one compartment is filled to a desired volume, and subsequently the unit is moved further such that the filled compartment is transferred into the treatment space.

According to a preferred embodiment of the present invention, the cell material is removed from the at least one compartment by means of an emptying unit. Any unit capable of removing cell material from the compartment can be used. Preferably, the emptying unit is a pipeline having an inlet in a region of the device remote from the treatment space, here designated as emptying region. When the at least one compartment filled with the treated cell material, after treatment in the treatment space, is moved into the emptying region, the compartment can be connected with the inlet of the emptying unit, for example by opening the bottom of the compartment.

The emptied compartment can be subsequently moved again into the filling region, to start another treatment cycle.

According to a second embodiment, the unit is a plate arranged on a motor for generating a rotational movement, on which plate the at least one container can be fastened.

Such units are known. To prevent that the container does not fall off the unit during the, preferably rotational, movement, the unit has means for fastening one or preferably a plurality of containers. Such means are known. They can, for example, be depressions in the surface of the unit or fastening devices provided on the surface of the unit, such as clamps or closures (screw caps, elastic closures, magnetic closures or the like).

It is preferred according to this embodiment that 1 to 20, preferably 2 to 15, more preferably 5 to 10 containers can be arranged on the unit.

According to a preferred embodiment of the present invention, the at least one container is filled with cell material by means of a filling unit. Said filling unit comprises an outlet through which cell material can be inserted into the at least one container.

The outlet of the filling unit is preferably connected to a device in which the cell material or a suspension containing the cell material is located. For example, conventional pipelines can be provided for this connection. Preferably, a shut-off unit such as a valve or a lock is provided in or at the outlet or alternatively in the connection described above in order to be able to control the introduction of the cell material or the suspension containing the cell material into the container.

The outlet of the filling unit is preferably located in a region of the device remote from the treatment space, here designated as filling region. Preferably, the filling region is arranged such that the unit is first moved into the filling region, where the at least one container is filled to a desired volume, and subsequently the unit is moved further such that the filled container is transferred into the treatment space.

Alternatively, an already filled container may be provided on the unit before the unit is moved to transport the filled container into the treatment space.

According to a preferred embodiment of the present invention, the cell material is removed from the at least one container by means of an emptying unit. Any unit capable of removing cell material from the container can be used. Preferably, the emptying unit is a pipeline having an inlet in a region of the device remote from the treatment space, here designated as emptying region. When the at least one container filled with the treated cell material, after treatment in the treatment space, is moved into the emptying region, the container can be connected with the inlet of the emptying unit.

Alternatively, the filled container may be removed from the unit altogether and replaced by an empty container (if the container is filled in the filling region) or by a filled container.

The empty container can be subsequently moved again into the filling region, to start another treatment cycle. Optionally, a sterilization/sanitization step may be performed prior to the start of another treatment cycle. Known sterilization/ sanitization processes such as steam-based processes can be used in accordance with the present invention.

The present invention further relates to a method for treating cells for stimulating cell growth, preferably performed in a device according to the present invention, comprising the steps
a) providing cell material in at least one compartment of a unit or at least one container on a unit,
b) applying an electric field to a treatment space,
c) performing a movement, preferably a rotational movement, of the unit into the treatment space.

The method can be performed as already described above.

As stated above, it is preferred that the cell material is provided as a suspension in an electrically conductive liquid in the at least one container.

As stated above, it is further preferred that the, preferably rotational, movement of the container or compartment into the treatment space is realized by arranging the at least one container or compartment on or in a rotatable unit and rotating this unit. The rotating unit can be a plate arranged on a motor for generating a rotational movement on which plate the at least one container can be fastened. The rotating unit can alternatively be a body, preferably a cylindrical body, which has at least one compartment for receiving the cell material and is arranged on a motor for generating a rotational movement.

As stated above, it is further preferred that an electric field is applied with such electric pulses, so that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

As stated above, the method is preferably carried out as follows. First, the unit is moved such that a respective compartment or container in or on said unit is moved into a filling region, where it is filled with cell material through a filling unit, as described above. Alternatively, an already filled container may be provided on said unit.

Subsequently, the unit is moved again such that the filled compartment or container enters the treatment space. When the filled compartment or container has been moved into the treatment space, movement of the unit is stopped for a period of treatment of the cell material that is present in the treatment space.

After the treatment has been performed, the filled compartment or container is moved, by movement of the unit, into an emptying region where the treated cell material is removed from the filled compartment or container, as described above. Alternatively, a filled container can be removed from the unit altogether.

The empty container or compartment can be subsequently moved again into the filling region, to start another treatment cycle. Optionally, a sterilization/sanitization step may be performed prior to the start of another treatment cycle. Known sterilization/ sanitization processes such as steam-based processes can be used in accordance with the present invention.

The present invention further relates to the use of the device according to the present invention described here for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe. 2019.00265.

The present invention is explained below by way of non-limiting examples and figures. Shown are:
- Fig. 1: a schematic representation of a first embodiment of the device of the present invention
- Fig. 2: a schematic representation of a second embodiment of the device of the present invention

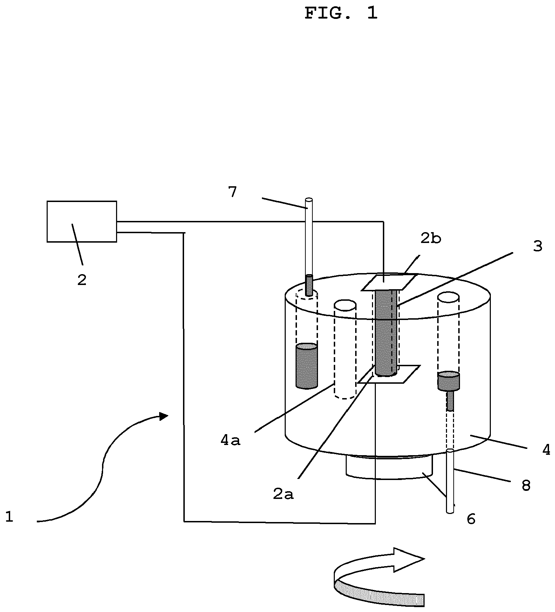

Fig. 1 shows a schematic representation of an embodiment of the device (1) of the present invention.

The device has a unit (2) for generating and emitting electric pulses, for example, a pulse generator. The unit (2) is electrically connected to two electrodes (2a, 2b). A treatment space (3) is located between and closed by the electrodes (2a, 2b), on which treatment space (3) electric pulses generated by the unit (2) are applied. The electrodes (2a, 2b) are arranged perpendicularly to the direction of movement of the unit (4). When the compartment (4a) enters the treatment space (3), it takes a position in the space between said electrodes (2a, 2b), so that one electrode (2a) is provided over said compartment (4a), and the other electrode (2b) is provided below said compartment (4a) .

A rotatable unit (4) is provided in the device (1). In the embodiment according to Fig. 1, this unit (4) is a cylindrical body, which by way of example here has 4 compartments (4a) for receiving cell materials. The compartments (4a) are blind holes of suitable dimension.

The rotatable unit (4) is arranged on a motor (6) which generates a rotational movement and sets the unit (4) arranged on it into rotary movement. The compartments (4a) also undergo a rotational movement in this way. Preferably, the unit (4) is rotated at least once around an imaginary axis defined through the center of the unit (4), whereby the compartments (4a) undergo a rotation of 360°. A multiple complete rotation of the unit (4) is possible and advantageous depending on the cell material to be treated.

In a region of the device (1) preceding the treatment space (3) in the direction of movement of the unit (4), a compartment (4a) is filled with cell material by means of a filling unit (7).

The filling unit (7) is connected to a device (not shown) in which the cell material or a suspension containing the cell material is located.
The filling region is arranged such that the compartment (4a) is first moved into the filling region, where the compartment (4a) is filled to a desired volume (during which time the compartment (4a) remains in a filling position relative to the filling unit (7)), and subsequently the unit (4) is moved further such that the filled compartment (4a) is transferred into the treatment space (3).

Subsequently, the filled compartment (4a) is transported into the treatment space (3), by means of movement of the unit (4). Therein, the cell material in the compartment (4a) undergoes the desired treatment.

After treatment, the cell material is removed from the compartment (4a) by means of an emptying unit (8). In the embodiment of Fig. 1, the emptying unit (8) is a pipeline having an inlet in an emptying region. When the at least one compartment (4a) filled with the treated cell material, after treatment in the treatment space (3), is moved into the emptying region, by means of movement of the unit (4), the compartment (4a) can be connected with the inlet of the emptying unit (8).

The empty compartment (4a) can be subsequently moved again into the filling region, to start another treatment cycle. Optionally, a sterilization/sanitization step may be performed prior to the start of another treatment cycle. Known sterilization/ sanitization processes such as steam-based processes can be used in accordance with the present invention.

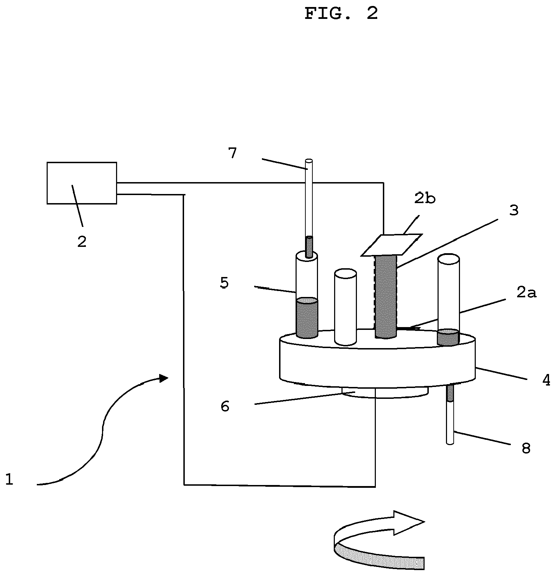

Fig. 2 shows a schematic representation of a second embodiment of the device (1) of the present invention. Like reference numerals designate the same components as in Fig. 1.

In this embodiment, a unit (4) in the form of a plate is provided. Said unit (4) comprises recesses in which one or more containers (5) can be securely arranged so as to not fall off during movement of the unit (4). In the embodiment according to Fig. 2, the unit (4) comprises 4 recesses and thus can take up 4 containers (5).

Similar to the embodiment according to Fig. 1, in a region of the device (1) preceding the treatment space (3) in the direction of movement of the unit (4), a container (5) is filled with cell material by means of a filling unit (7).

The filling unit (7) is connected to a device (not shown) in which the cell material or a suspension containing the cell material is located.

The filling region is arranged such that the container (5)is first moved into the filling region, where the container (5) is filled to a desired volume (during which time the container (5) remains in a filling position relative to the filling unit (7)), and subsequently the unit (4) is moved further such that the filled container (5) is transferred into the treatment space (3) .

Alternatively, instead of filling a container (5) in the filling region, also a filled container (5) may be put (manually or automatically) into a free recess on the unit (4).

Subsequently, the filled container (5) is transported into the treatment space (3), by means of movement of the unit (4). Therein, the cell material in the container (5) undergoes the desired treatment.

After treatment, the cell material is removed from the container (5) by means of an emptying unit (8). In the embodiment of Fig. 2, the emptying unit (8) is a pipeline having an inlet in an emptying region. When the at least one container (5) filled with the treated cell material, after treatment in the treatment space (3), is moved into the emptying region, by means of movement of the unit (4), the container (5) can be connected with the inlet of the emptying unit (8).

Alternatively, instead of emptying a container (5) in the emptying region, also a filled container (5) may be removed (manually or automatically) from the unit (4) altogether.

The empty container (5) can be subsequently moved again into the filling region, to start another treatment cycle. Alternatively, an empty or filled container (5) may be put into a free recess on the unit (4).

## Claims

1. A device (1), comprising a unit (2) for generating and emitting electric pulses, and a treatment space (3) that can be penetrated by the emitted electric pulses and an electric field resulting therefrom, **characterized in that** the device (1) further comprises a unit (4) which can be moved into and out of said treatment space (3) such that cell material provided within a compartment (4a) of said unit (4), or provided in a container (5) on said unit (4), can be moved into and out of said treatment space (3).

2. The device according to claim 1, **characterized in that** the unit (4) is provided on a motor (6) for generating a rotational movement.

3. The device according to claim 2, **characterized in that** the unit (4) is a plate arranged on said motor (6) for generating a rotational movement, on which plate the at least one container (5) can be fastened.

4. The device according to claim 3, **characterized in that** 1 to 20, preferably 2 to 15, more preferably 5 to 10 containers (5) can be arranged on the unit (4).

5. The device according to claim 2, **characterized in that** the unit (4) is a body, preferably a cylindrical body, which has at least one compartment (4a) for receiving cell material and is arranged on said motor (6) for generating a rotational movement.

6. The device according to claim 5, **characterized in that** 1 to 20, preferably 2 to 15, more preferably 5 to 10 compartments (4a) are arranged in the unit (4).

7. The device according to any one of claims 1 to 6, **characterized in that** the device (1) furthermore comprises a filling unit (7) for filling cell material into the at least one compartment (4a) or the at least one container (5) .

8. The device according to any one of claims 1 to 7, **characterized in that** the device (1) furthermore comprises an emptying unit (7) for removing cell material from the at least one compartment (4a) or the at least one container (5) .

9. The device according to any one of claims 1 to 8, **characterized in that** the unit (2) for generating and emitting electric pulses comprises two electrodes (2a, 2b) or plates of a capacitor and can generate electric pulses, so that a voltage increase take place between the two electrodes (2a, 2b) of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

10. A method for treating cells for stimulating cell growth, preferably performed in a device (1) according to any one of claims 1 to 9, comprising the steps
a) providing cell material in at least one compartment (4a) of a unit (4) or at least one container (5) on a unit (4),
b) applying an electric field to a treatment space (3),
c) performing a movement, preferably a rotational movement, of the unit (4) into the treatment space (3).

11. The method according to claim 10, **characterized in that** the cell material is provided in the at least one compartment (4a) of the unit (4) or at least one container (5) on the unit (4) as a suspension in an electrically conductive liquid.

12. The method according to claim 10 or 11, **characterized in that** the cell material is provided in the at least one compartment (4a) of the unit (4) or at least one container (5) on the unit (4) through a filling unit (7).

13. The method according to any one of claims 10 to 12, **characterized in that** a rotational movement of the at least one compartment (4a) of the unit (4) or at least one container (5) on the unit (4) into the treatment space (3) is realized by moving the unit (4) through the treatment space (3), preferably by performing a rotational movement of the unit (4).

14. The method according to any one of claims 10 to 13, **characterized in that** when the unit (4) has been moved into the treatment space (3), movement of the unit (4) is stopped for a period of treatment of the cell material that is present in the treatment space (3).

15. The method according to any one of claims 10 to 14, **characterized in that** an electric field is applied with such electric pulses, so that a voltage increase takes place between the two electrodes (2a, 2b) or plates of a capacitor of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.
